# EUROPÄISCHE PATENTSCHRIFT

(11) **EP 0 844 992 B1**
(45) Veröffentlichungstag und Bekanntmachung des Hinweises auf die Patenterteilung: **24.05.2000**
(21) Anmeldenummer: 96921989.8
(22) Anmeldetag: 14.06.1996
(51) Int. Cl.: C07C 231/12, C07C 227/26, C07D 305/14

(54) **VERFAHREN ZUR HERSTELLUNG VON BETA-AMINO-ALPHA-HYDROXYCARBONSÄUREN UND DEREN DERIVATEN**
PROCESS FOR PRODUCING BETA-AMINO-ALPHA-HYDROXYCARBOXYLIC ACIDS AND DERIVATIVES THEREOF
PROCEDE POUR LA PREPARATION D'ACIDES BETA-AMINO-ALPHA-HYDROXYCARBOXYLIQUES ET DE LEURS DERIVES

(30) Priorität: 04.07.1995 DE 19524337
(43) Veröffentlichungstag der Anmeldung: 03.06.1998
(73) Patentinhaber: Degussa-Hüls Aktiengesellschaft, 60287 Frankfurt am Main (DE)
(72) Erfinder: STINGL, Klaus, D-63755 Alzenau (DE); KOTTENHAHN, Matthias, D-63579 Freigericht (DE); DRAUZ, Karlheinz, D-63579 Freigericht (DE)
(86) Internationale Anmeldenummer: EP9602573
(87) Internationale Veröffentlichungsnummer: WO9702236

(56) Entgegenhaltungen:
- US-A- 5 420 337
- TETRAHEDRON LETT. (1994), 35(18), 2845-8 CODEN: TELEAY;ISSN: 0040-4039, 1994, XP000605432 KEARNS, JEFF ET AL: "Application of yeast-catalyzed reductions to synthesis of (2R,3S)-phenylisoserine" in der Anmeldung erwähnt
- SYNTHESIS (1995), (2), 181-6 CODEN: SYNTBF;ISSN: 0039-7881, 1995, XP002018357 DONDONI, ALESSANDRO ET AL: "Synthesis of Taxol and Taxotere side chains by 2- (trimethylsilyl)thiazole based homologation of L-phenylglycine"

## Beschreibung

Die Erfindung betrifft ein neues Verfahren zur Herstellung von β-Amino-α-hydroxy-carbonsäuren und Derivate der allgemeinen Struktur (2R,3S)- oder (2S,3R)-N-(X,Y)-3-Amino-2-hydroxy-3-phenyl-propionsäure-Z der Formel I 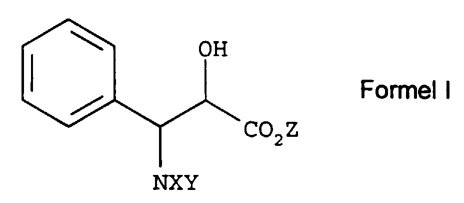 worin
- X: H, (C₁-C₆) Alkyl, Benzyl,
- Y: (C₁-C₆) Alkyl, Benzyl, Formyl, COR¹ oder CO₂R²,
- X,Y: zusammen Phthaloyl, Maleinoyl oder Maloneyl,
- R¹: (C₁-C₆) Alkyl, Phenyl, Benzyl, NH₂, 4-NO₂-Phenyl oder 4-NO₂-Benzyl,
- R²: (C₁-C₆) Alkyl, Phenyl, Benzyl, 4-NO₂-Phenyl oder 4-NO₂-Benzyl,
- Z: H, (C₁-C₅) Alkyl, Phenyl, Benzyl, 4-NO₂-Benzyl, 4-NO₂-Phenyl oder Allyl
bedeuten.

Verbindungen des Typs I sind wertvolle Zwischenstufen bei der Totalsynthese für Taxol (Paclitaxel), das zur Behandlung von diversen Krebsarten Einsatz findet.

Eine unter Formel I fallende Verbindung (2R,3S)-N-Benzoyl-3-amino-2-hydroxy-3-phenylpropionsäuremethylester der Formel II 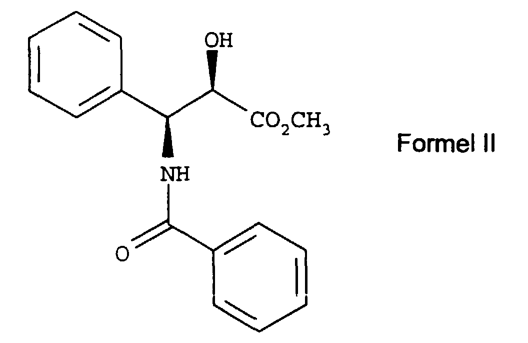 ist beispielsweise in Tetrahedron Letters 35, 2845 (1994) ebenfalls ausgehend von optisch aktivem Phenylglycin beschrieben und hat als einen Schlüsselschritt die enzymatische Reduktion eines α-Ketoester. Ein wesentlicher Nachteil dieser Synthesemethode ist, daß die Verbindung der Formel II in lediglich 12 % Ausbeute isoliert werden kann. Aufgabe der vorliegenden Erfindung ist demnach, ein Verfahren zur Verfügung zu stellen, das höhere Ausbeuten liefert und dabei umweltfreundlich und ökonomisch abläuft.

Das erfindungsgemäße Verfahren geht von
a) (S)- oder (R)-Phenylglycin der Formel III 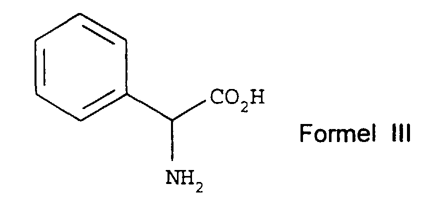 als Ausgangssubstanz aus. Diese wird mit Hydridreagenzien reduziert.
   Geeignete Hydridreagenzien sind Lithiumaluminiumhydrid oder Natriumborhydrid/Aktivator.
b) Das in Schritt (a) erhaltene (S)- oder (R)-Phenylglycinol der Formel IV 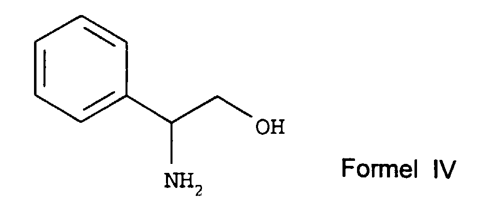 wird in den
c) N-geschützten β-Aminoalkohol (S)- oder (R)-N-(X,Y)-Phenylglycinol der allgemeinen Formel V 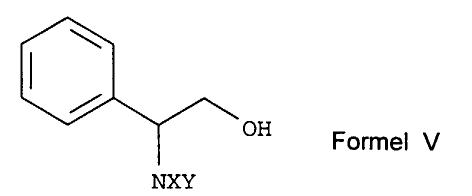 überführt, worin
   - X: H, (C₁-C₆) Alkyl oder Benzyl,
   - Y: (C₁-C₆) Alkyl, Benzyl, Formyl, COR¹ oder CO₂R²,
   - XY: zusammen Phthaloyl, Maleinoyl oder Maloneyl,
   - R¹: (C₁-C₆) Alkyl, Phenyl, Benzyl, NH₂, 4-NO₂-Phenyl oder 4-NO₂-Benzyl,
   - R²: (C₁-C₆) Alkyl, Phenyl, Benzyl, 4-NO₂-Phenyl, 4-NO₂-Benzyl oder Allyl
   bedeuten.
   Diese vorübergehende Blockierung der reaktiven Aminogruppe vor der anschließenden Oxidation der Hydroxylgruppe ist zu deren Erhaltung notwendig und erfolgt, wie später noch detalliert beschrieben.
d) Durch Oxidation von Verbindungen der Formel V wird ein (S)- oder (R)-N-(X,Y)-Phenylglycinal der Formel VI erhalten. 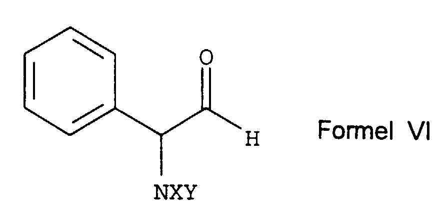
   Die Oxidation, worin X und Y die oben genannten Bedeutungen besitzen, erfolgt, wie später detalliert beschrieben. Anschließend wird die Verbindung der Formel VI
e) zum (1RS,2S)- oder (1RS,2R)-2-(X,Y)-Amino-1-cyano-2-phenylethan-1-ol der Formel VII 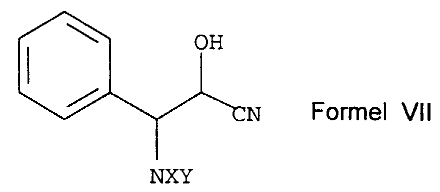 worin X und Y die oben erwähnten Bedeutungen haben, umgesetzt. Die Herstellung des Nitrils kann mit an sich bekannten Verfahren erfolgen.
f) Das erhaltene Nitril (Formel VII) wird zu den Säuren VIII + IX oder auch ihrer Additionssalze der Formel X und XI 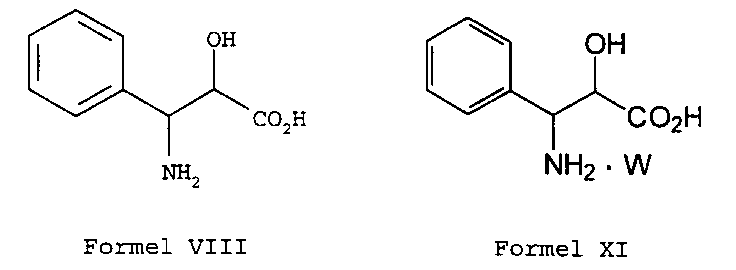 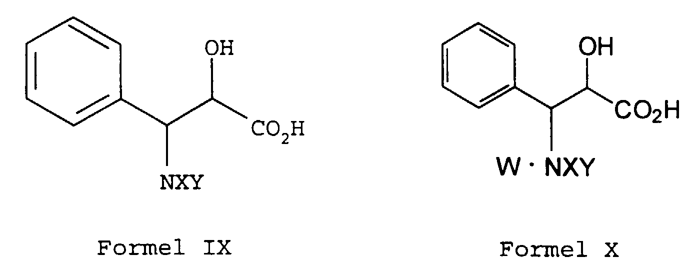 hydrolysiert, worin X und Y die oben angegebene Bedeutung haben und W für HCl, HBr oder H₂SO₄ steht. Die Hydrolyse wird vorzugsweise, wie später detalliert erläutert, ausgeführt.
g) Anschließend werden die Verbindungen der Formeln VIII und XI nach üblichen Methoden zum (2RS,3S)- oder (2RS,3R)-3-Amino-2-hydroxy-3-phenylpropionsäureester der Formel XII 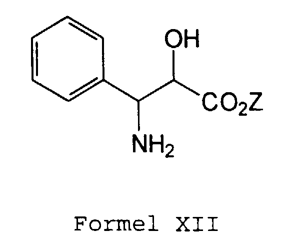 umgesetzt, wobei Z gleich (C₁-C₆) Alkyl, Phenyl, Benzyl, 4-NO₂-Phenyl, 4-NO₂-Benzyl oder Allyl bedeutet, und
i) anschließend wird die noch freie Stickstoffunktion der Formel X nach ebenfalls bekannten Methoden zu Verbindungen der Formel I 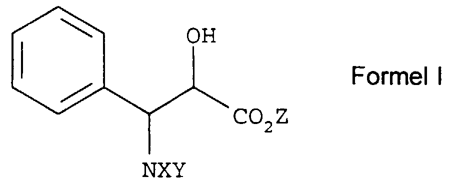 geschützt, wobei Z, X und Y die oben angegebene Bedeutung besitzen,
   oder
k) man setzt die Verbindungen IX und X wie unter g) beschrieben zu Verbindungen der Formel I um.

(S)- oder (R)-Phenylglycinol V erhält man nach an sich bekannten Methoden, in dem man die Säurefunktion von z. B. (S)-Phenylglycin III gemäß JP-OS-5-221935 mit Hydridreagenzien, wie beispielsweise Lithiumaluminiumhydrid oder Natriumborhydrid/Aktivator, bevorzugt mit dem System Natriumborhydrid/Schwefelsäure zum (S)-Phenylglycinol IV reduziert.

Die Blockierung der Stickstoffunktion des β-Aminoalkohols (R)- oder (S)-Phenylglycinol V wird ebenfalls in an sich bekannter Weise (analog Synth. Commun. 1995, 25, S. 561) durchgeführt. So wird z. B. die Reaktionslösung aus der vorherigen Reaktionsstufe bei pH = 6 - 12, vorteilhafterweise bei pH = 7 - 8.5, und 0 - 15 °C mit z. B. einem Chlorameisensäure-niederalkylester versetzt. Die Verbindung kann durch Kristallisation aus z. B. Toluol gereinigt werden, indem man den carbamoylierten β-Aminoalkohol aus der alkalischen Reaktionslösung in heißes Toluol extrahiert.

Die Oxidation des N-geschützten Aminoalkohols V zum korrespondierenden N-blockierten Aminoaldehyd VI wird ebenfalls nach an sich bekannten Methoden (analog Tetrahedron Letters 33, 5029 (1992)) durchgeführt. Dazu wird die Vorstufe V durch Oxidation mit Hilfe von TEMPO (2,2,6, 6-Tetramethylpiperidin-l-oxyl) in Gegenwart von Hypochlorit-Lösung [NaOCl oder NaOBr (aus NaOCl und KBr generiert)] in den Aminoaldehyd überführt. Dabei werden von dem Nitroxyradikal (TEMPO) Mengen im Bereich von 0.05 - 10 mol%, vorteilhaft 0.5 - 2 mol%, von Hypochlorit-Lösung [NaOCl oder NaOBr (aus NaOCl und KBr generiert), normalerweise 12 - 13 %ig] 1 - 4 Äquivalente, vorteilhaft 1.2 Äquivalente, verwendet. Beide Reagenzien sind selbst im technischen Maßstab einfach zu handhaben und sind kommerziell erhältlich. Da für die Anwendbarkeit des Oxidationssystems die Anwesenheit von hypochloriger-Säure oder hypobromiger-Säure Voraussetzung ist, wird diese aus technischer Chlorbleichlauge oder technischer Chlorbleichlauge und KBr durch pH-Wert Herabsetzung mit Säure oder einer pH-Wert senkenden Base, vorteilhafterweise, wie z. B. Natriumhydrogencarbonat, in situ generiert. Die Oxidationsreaktion verläuft bei Temperaturen von -30 - 15 °C, besonders vorteilhaft zwischen -20 und -10 °C in einem Zweiphasensystem sehr effektiv. Als geeignete Lösungsmittel erwiesen sich grundsätzlich alle inerten organischen Lösungsmittel. Insbesondere jedoch sind halogenierte Kohlenwasserstoffe, wie zum Beispiel Dichlormethan, Chloroform, 1,1,1-Trichlorethan oder auch Ester der Ameisensäure und Essigsäure von Vorteil. Zur Vernichtung von überschüssiger hypochloriger Säure wird mit einem Reduktionsmittel, wie z. B. Natriumthiosulfatlösung aufgearbeitet. Dieser Oxidationsschritt ist schwermetallfrei und somit als besonders umweltschonend einzustufen.

Zur Herstellung des Nitrils der Formel VII wird in an sich bekannter Weise eine Lösung des Aminoaldehyds VI in einem inerten organischen Lösungsmittel, bevorzugt in Toluol, Dichlormethan oder Essigsäureethylester mit 1 - 5 Äquivalenten Cyanwasserstoff, vorteilhafterweise 1 - 1.3 Äquivalenten und Zusatz von 10 - 0.01 mol% einer Base, bevorzugt 0.1 - 0.05 mol% einer tertiären organischen Stickstoffbase, wie z. B. Triethylamin, N-Methylmorpholin oder Tributylamin, bei Temperaturen zwischen 0 und +35 °C, bevorzugt bei +15 °C versetzt. Überraschenderweise verläuft die Cyanwasserstoffaddition mit unerwartet hoher Stereoselektivität. Beim Einsatz von (S)-Phenylglycin werden Diastereoselektivitäten von (1R,2S):(1S,2S) zwischen 65:35 bis zu >74:26 beobachtet.

Die Hydrolyse des Cyanhydrins VII mit einer anorganischen Säure, beispielsweise einer 21 % Salzsäure unter Rückflußtemperatur zu den Hydroxyaminosäuren VIII und XI erfolgt ebenfalls in an sich bekannter Weise (analog Liebigs Annalen der Chemie 749, 198 (1971)). Zur Abtrennung von bei der Hydrolyse entstandenen Crack-Produkten ist es von Vorteil die salzsaure Lösung mit Toluol oder einem anderen unter diesen Bedingungen inerten Lösungsmittel zu waschen. Zur Isolation von Verbindungen der allgemeinen Formel VIII muß anschließend mit Hilfe einer anorganischen Base, wie beispielsweise NaOH, KOH, NaHCO₃ oder Na₂CO₃, auf einen pH-Wert von 4 - 8, vorzugsweise 6,5 - 7, eingestellt werden. Im Rahmen dieser Erfindung erwies sich die unter diesen Bedingungen gleichzeitige Abspaltbarkeit der Schutzgruppe von Vorteil für den weiteren Syntheseverlauf. Prinzipiell ist es jedoch auch denkbar, daß N-Schutzgruppen eingesetzt werden, die unter den CN-Hydrolysebedingungen direkt Verbindungen der allgemeinen Formel I mit Z = H ergeben (Formeln IX und X).

Desweiteren wurde gefunden, daß eine Zwischenisolierung von XI als Hydrochlorid durch schlichtes Einengen der salzsauren Reaktionslösung, neben einer hohen chemischen Reinheit ebenfalls eine unerwartet hohe Diastereomerenreinheit [bei Einsatz von (S)-Phenylglycin (2R,3S):(2S,3S) zwischen 90:10 bis zu >95:5] dieser Verbindung zur Folge hat. Mit ebenso hoher chemischer wie diastereomerer Reinheit [bei Einsatz von (S)-Phenylglycin dv (2R,3S):(2S,3S) zwischen 90:10 bis zu >95:5] wird die Säure VIII aus der salzsauren Reaktionslösung durch pH-Wert Erhöhung auf 4 - 8, bevorzugterweise 6.5 - 7.0 erhalten.

Die Esterbildung wird nach an sich bekannten Methoden in Gegenwart von 1.1 - 4 Äquivalenten einer anorganischen Säure, vorteilhafterweise mit 1.1 Äquivalenten Schwefelsäure durchgeführt.

Eine Benzoylierung des z. B. eines Methylesters der Formel XII mit Z = CH₃ erfolgt nach an sich bekannter Weise, indem man eine wäßrige Lösung bzw. Suspension des Esters XII mit Natriumhydrogencarbonat puffert und bei Temperaturen zwischen +5 bis +15 °C mit Benzoylchlorid versetzt.

In einer anderen Variante wird die N-geschützte α-Hydroxysäure IX dadurch hergestellt, daß man
a) die (2RS,3S)- oder (2RS,3R)-3-Amino-2-hydroxy-2-phenylpropionsäure der Formel VIII 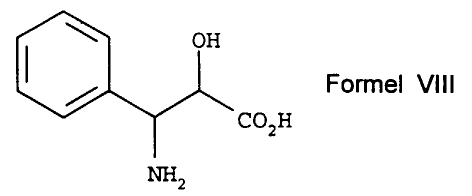 zunächst mit einem Schutzgruppenreagenz in die N-geschützte Form der Formel IX 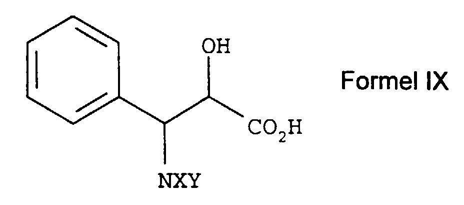 umwandelt, wobei X und Y die oben angegebene Bedeutung haben,
b) und mit einem Veresterungsreagenz zum Ester der Formel I 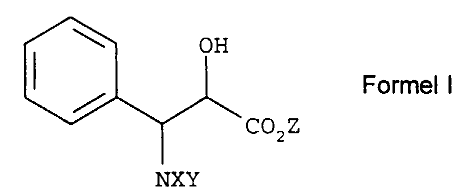 umsetzt, wobei X, Y und Z die oben angegebene Bedeutung besitzen.

Eine Benzoylierung von z. B. (2RS,3S)-2-Amino-3-hydroxy-2-phenylpropionsäure VIII erfolgt nach an sich bekannter Weise, indem man eine wäßrige Lösung oder Suspension der Säure VIII unter Basenzusatz, beispielsweise einer anorganischen Base, wie NaOH, KOH, NaHCO₃ oder Na₂CO₃, oder einer organischen Base, wie Triethylamin oder N-Methylmorpholin, bei einer Temperatur von +5 - +15 °C, vorteilhafterweise bei +5 °C und einem pH-Bereich von 6 - 12, vorteilhaft bei pH = 9.0 mit Benzoylchlorid versetzt.

Die Veresterung einer z. B. benzoylierten α-Hydroxysäure IX (R¹ = H, R² = COR³ und R³ = Phenyl) wird zweckmäßigerweise in Gegenwart einer katalytischen Menge einer Säure, wie beispielsweise Schwefelsäure, unter Erhitzen, z. B. bis auf Rückflußtemperatur, durchgeführt.

Die folgenden Beispiele sollen die vorliegende Erfindung erläutern, sie jedoch in keiner Weise einschränken. Alle Temperaturen sind in Celciusgraden angegeben.

### Beispiel 1:

### (S)-N-Methoxycarbonyl-phenylglycinol

Zu einer Suspension von 75.6 g (0.5 mol) (S)-Phenylglycin und 47.5 g NaBH₄ in 750 ml DME (Dimethoxyethan) tropft man bei +5 °C eine Lösung von 33 ml (0.625 mol) konz. H₂SO₄ und 120 ml DME innerhalb von ca. 1 h so zu, daß +10 °C nicht überschritten werden. Nach beendeter Zugabe läßt man die Mischung auf Raumtemperatur kommen und erhitzt vorsichtig für 2 h unter Rückfluß. Anschließend destilliert man ca. ¾ des verwendeten Lösungsmittel ab, quentscht mit 100 ml MeOH, 200 ml H₂O und 50 ml konz. HCl in der angegebenen Reihenfolge zwischen +10 und +30 °C. Mit 50 %iger NaOH stellt man einen pH-Wert von 8 ein und tropft bei +5 °C 42.3 ml (0.55 mol) Methylchlorformiat so zu, daß +15 °C nicht überschritten werden. Der pH wird währenddessen mit 50 %iger NaOH zwischen 7.5 und 8.5 gehalten und rührt nach beendeter Zugabe des Carbamoylierungmittels noch ca. 1 h nach. Anschließend fügt man 500 ml Toluol hinzu, erwärmt auf +50 °C, trennt die Phasen und extrahiert die alkalische Phase erneut bei der oben angegebenen Temperatur mit 300 ml Toluol. Die vereinigten organischen Auszüge werden bis auf 200 ml eingeengt und das ausgefallene farblose Produkt bei 50 °C im Vakuumtrockenschrank getrocknet.
Ausbeute: 76.2 g (78 %).
Reinheit: > 95 % nach 1H-NMR.
Smp.: 102 - 105 °C.

| | | | | |
|---|---|---|---|---|
| C10H13NO3 | ber. | C 61.53 | H 6.71 | N 7.17 |
| | gef. | C 61.62 | H 6.93 | N 7.34. |

### Beispiel 2:

### (S)-N-Ethoxycarbonyl-phenylglycinol

Durchführung der Synthese und Ansatzgröße analog Beispiel 1. Als Carbamoylierungsreagenz wird jetzt Ethylchlorformiat verwendet.
Ausbeute: 89.0 g (85 %).
Reinheit: >95 % nach ¹H-NMR.
Smp.: 90 - 93 °C.

| | | | | |
|---|---|---|---|---|
| C11H15NO3 | ber. | C 63.14 | H 7.23 | N 6.69 |
| | gef. | C 63.19 | H 7.43 | N 7.05. |

### Beispiel 3:

### (2RS,3S)-3-Amino-2-hydroxy-3-phenylpropionsäure

Zu einer auf -17 °C gekühlten Suspension von 180.2 g (861 mmol) (S)-N-Ethoxycarbonyl-phenylglycinol in 1.5 l Essigsäureethylester fügt man 10 g (84 mmol) KBr und 235 mg (1.5 mmol) TEMPO (2,2,6,6-Tetramethylpiperidin-1-oxyl). Dazu tropft man eine auf pH 8.9 - 9.2 eingestellte NaOCl-Lösung [665 g techn. NaOCl (12 - 13 %ig), 400 ml H₂O und ca. 115 g NaHCO₃] innerhalb von 3 h so zu, daß -12 °C nicht überschritten werden. Nach Zugabe von 350 ml einer 5 %igen Natriumthiosulfat-Lösung und 100 ml H₂O trennt man die Phasen und extrahiert die wäßrige einmal mit 500 ml Essigsäureethylester. Die vereinigten organischen Extrakte werden einmal mit 400 ml ges. NaHCO₃-Lösung und 200 ml H₂O gewaschen. Nach Trocknen mit MgSO₄ gibt man 70 mg (0.7 mmol) Triethylamin hinzu, kühlt auf +15 °C und tropft 31 ml (800 mmol) HCN so zu, daß die Temperatur +25 °C nicht übersteigt und rührt nach beendeter Zugabe noch 2 h nach. Nun fügt man zu der Reaktionslösung 1.3 1 einer 21 %igen wäßrigen Salzsäure, strippt den Essigsäureethylester ab und erhitzt für 16 h unter Rückfluß und extrahiert die abgekühlte (RT) salzsaure Lösung einmal mit 300 ml Toluol. Anschließend wird die salzsaure Lösung auf ein Volumen von ca. 300 ml eingeengt und mit 40 %iger NaOH ein pH-Wert von 6.8 bei einer Temperatur von +15 - 25 °C eingestellt. Die Mischung wird auf +5 °C gekühlt, das ausfallende Produkt abgesaugt, mit 200 ml kaltem H₂O nachgewaschen und bei 50 °C im Vakuumtrockenschrank getrocknet. Man erhält so das Produkt in Form eines gelben Feststoffs.
Ausbeute: 90 g, davon 33 % NaCl, also 60.3 g (39 %).
Reinheit: 94 % nach HPLC.
dv [(2R,3S):(2S,3S)]: 92:8 per HPLC bestimmt.

### Beispiel 4:

### (2R,3S)-N-Benzoyl-3-amino-2-hydroxy-3-phenylpropionsäuremethylester

Zu einer Suspension von 15.0 g (55.5 mmol, da nur 67 %ig) (2RS,3S)-2-Amino-3-hydroxy-2-phenylpropionsäure (aus Beispiel 3) in 250 ml Methanol tropft man vorsichtig bei Raumtemperatur 5 ml konz. H₂SO₄ und erhitzt 6 h unter Rückfluß. Man versetzt mit 50 ml H₂O und stellt mit 50 %iger NaOH bei +5 °C einen pH von 6.8 ein. Anschließend entfernt man das Methanol per Vakuumdestillation, kühlt auf +5 °C, versetzt mit 10 g (120 mmol) NaHCO₃ und tropft 8.4 ml (72 mmol) Benzoylchlorid innerhalb von 20 min. zu.

Nachdem man hat weitere 2 h rühren lassen, wird das ausfallende Rohprodukt abgesaugt, aus 150 ml Methanol umkristallisiert und bei 50 °C im Vakuumtrockenschrank zu einem farblosen Produkt getrocknet.
Ausbeute: 12.1 g (73 %).
Reinheit: >99 % nach HPLC.
Smp.: 162 - 168 °C.
dv [(2R,3S):(2S,3S)]: >95 % nach HPLC und ¹H-NMR.

### Beispiel 5:

### (2R,3S)-3-Amino-2-hydroxy-3-phenylpropionsäure-Hydrochlorid

Zu 60 ml einer 1 molaren Lösung von (1RS,2S)-N-Methoxycarbonyl-2-amino-1-cyano-2-phenylethan-1-ol in Dichlormethan gibt man 160 ml konz. HCl und destilliert Dichlormethan ab. Anschließend erhitzt man die Lösung 10 h unter Rückfluß und extrahiert die auf Raumtemperatur abgekühlte Reaktionslösung einmal mit 50 ml Toluol. Nach Einengen der salzsauren Lösung bis auf ca. 30 - 40 ml saugt man den ausgefallenen Feststoff ab, wäscht mit Ether nach und trocknet bei 50 °C im Vakuumtrockenschrank.
Ausbeute: 1.9 g (15 %).
Reinheit: >95 % nach ¹H-NMR.
dv [(2R,3S):(2S,3S)]: >95:5 nach ¹H-NMR.

### Beispiel 6:

### (2R,3S)-N-Benzoyl-3-amino-2-hydroxy-3-phenylpropionsäuremethylester

1.50 g (2R,3S)-2-Amino-3-hydroxy-2-phenylpropionsäure-Hydrochlorid (aus Beispiel 5) werden in 80 ml eines H₂O/Toluol-Gemisches (1:1) gelöst und bei einem pH-Wert von 9.0 mit 0.93 ml (8 mmol) Benzoylchlorid versetzt, wobei der pH-Wert mit 30 %iger NaOH nachgestellt wird. Nach beendeter Reaktion werden die Phasen getrennt, die alkalische Phase einmal mit 50 ml Toluol extrahiert und anschließend mit 2N HCl bei +5 °C auf pH = 2.0 gestellt. Der dabei ausfallende Feststoff wird abgesaugt und mit 100 ml Toluol koevaporiert. Dann löst man diesen in 80 ml MeOH, versetzt mit 0.1 ml konz. H₂SO₄ und erhitzt 4 h unter Rückfluß. Nachdem bis auf ca. 10 ml eingeengt wurde, verdünnt man mit 30 ml Diethylether, saugt das ausfallende farblose Produkt ab und trocknet bei 50 °C im Vakuumtrockenschrank. Ausbeute: 1.54 g (75 %).
Reinheit: >95 % nach HPLC.
dv [(2R,3S):(2S,3S)]: >95 % nach ¹H-NMR.

Allgemeine Arbeitsvorschrift zur Synthese von (1RS,2S)-N-Ethoxycarbonyl-2-amino-1-cyano-2-phenylethan-1-ol (Diastereomerenverhältnis in Abhängigkeit vom verwendeten Lösungsmittel), Beispiele 1 - 7:

Zu einer Lösung von 32 ml einer 10 %igen Aldehyd-Lösung (15.4 mmol) und 10 mg (0.1 mmol) Triethylamin tropft man bei 15 °C innerhalb von 10 min. 0.66 ml (17 mmol) HCN zu und rührt 30 min. bis zu 1 h nach. Anschließend wird der Umsatz und das Diastereomerenverhältnis (dv-Wert) per HPLC bestimmt.

### Beispiel 7:

Lösungsmittel: Dichlormethan.
Umsatz: quantitativ.
dv [(1R,2S):(1S,2S)]: 69:31.

### Beispiel 8:

Lösungsmittel: Toluol.
Umsatz: quantitativ.
dv [(1R,2S):(1S,2S)]: 73:27

### Beispiel 9:

Lösungsmittel: Essigsäureethylester.
Umsatz: quantitativ.
dv [(1R,2S):(1S,2S)]: 74:26.

## Patentansprüche

1. Verfahren zur Herstellung von β-Amino-α-hydroxy-carbonsäuren und Derivate der allgemeinen Struktur (2R,3S)- oder (2S,3R)-N-(X,Y)-3-Amino-2-hydroxy-3-phenyl-propionsäure-Z der Formel I 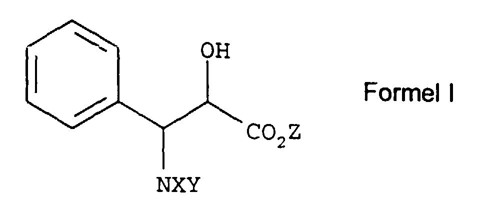 worin
X H, (C₁-C₆) Alkyl, Benzyl,
Y (C₁-C₆) Alkyl, Benzyl, Formyl, COR¹ oder CO₂R²,
X,Y zusammen Phthaloyl, Maleinoyl oder Maloneyl,
R¹ (C₁-C₆) Alkyl, Phenyl, Benzyl, NH₂, 4-NO₂-Phenyl oder 4-NO₂-Benzyl,
R² (C₁-C₆) Alkyl, Phenyl, Benzyl, 4-NO₂-Phenyl oder 4-NO₂-Benzyl,
Z H, (C₁-C₅) Alkyl, Phenyl, Benzyl, 4-NO₂-Benzyl, 4-NO₂-Phenyl oder Allyl
bedeuten,
dadurch gekennzeichnet, daß man
a) (S)- oder (R)-Phenylglycin der Formel III 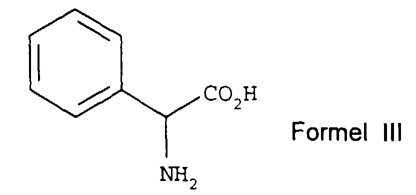 mit Hydridreagenzien reduziert,
b) das erhaltene (S)- oder (R)-Phenylglycinol der Formel IV 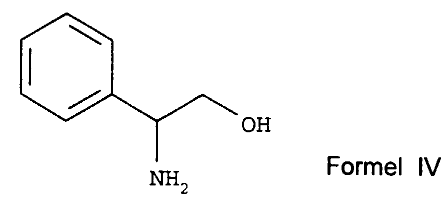 nach üblichen Methoden in den
c) N-geschützten β-Aminoalkohol (S)- oder (R)-N-(X,Y)-Phenylglycinol der allgemeinen Formel V 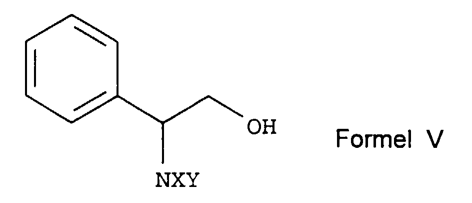 überführt, worin X und Y die oben angegebene Bedeutung haben,
d) das durch bekannte Oxidationsmethoden von Verbindungen der Formel V erhaltene (S)- oder (R)-N-(X,Y)-Phenylglycinal der Formel VI 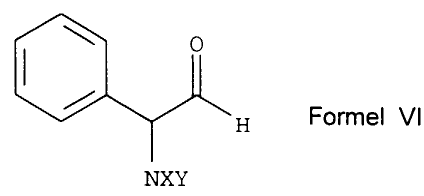 worin X und Y die oben genannten Bedeutungen besitzen,
e) in an sich bekannter Weise zum (1RS,2S)- oder (1RS,2R)-2-(X,Y)-Amino-1-cyano-2-phenylethan-1-ol der Formel VII 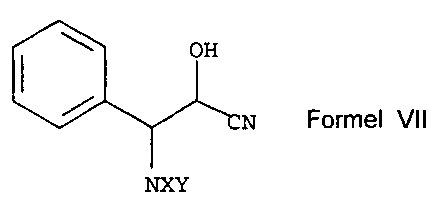 worin X und Y die oben erwähnten Bedeutungen haben, umsetzt,
f) das erhaltene Nitril (Formel VII) nach üblichen Methoden zu den Säuren VIII + IX oder auch ihrer Additionssalze der Formel X und XI 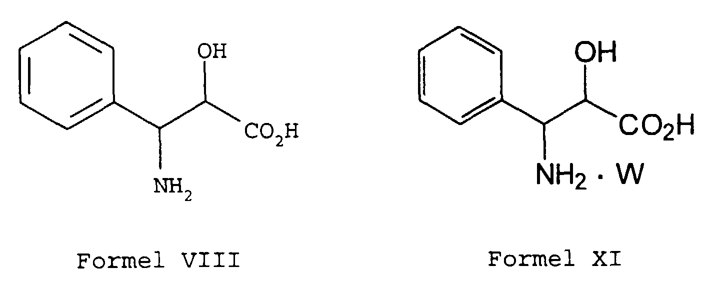 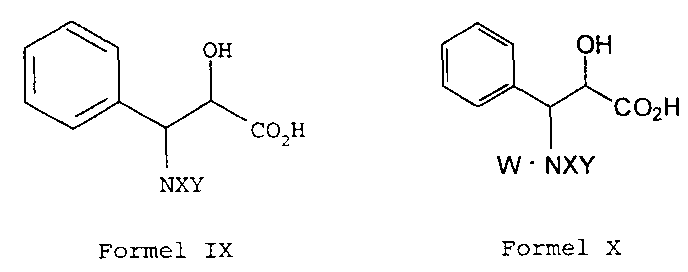 hydrolysiert, worin X und Y die oben angegebene Bedeutung haben und W für HCl, HBr oder H₂SO₄ steht,
g) anschließend die Verbindungen der Formeln VIII und XI nach üblichen Methoden zum (2RS,3S)- oder (2RS,3R)-3-Amino-2-hydroxy-3-phenylpropionsäureester der Formel XII 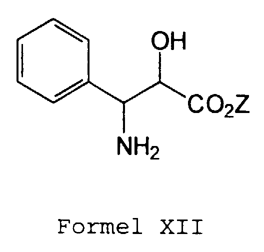 umsetzt, wobei Z gleich (C₁-C₆) Alkyl, Phenyl, Benzyl, 4-NO₂-Phenyl, 4-NO₂-Benzyl oder Allyl bedeutet, und
i) dann die noch freie Stickstoffunktion der Formel X nach ebenfalls bekannten Methoden schützt, was zu Verbindungen der Formel I 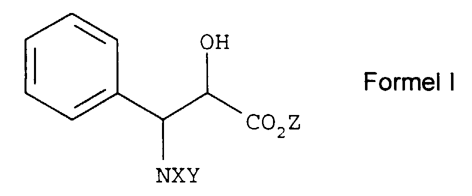 führt, wobei Z, X und Y die oben angegebene Bedeutung besitzen
oder daß
k) man die Verbindungen IX und X wie unter g) beschrieben zu Verbindungen der Formel I umsetzt,
bzw. daß man
l) die (2RS,3S)- oder (2RS,3R)-3-Amino-2-hydroxy-2-phenyl-propionsäure der Formel VIII 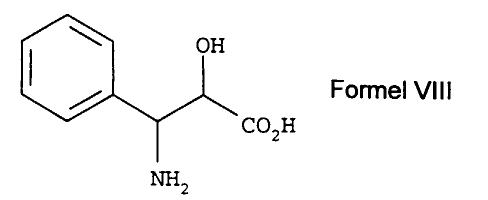 zunächst in üblicher Weise mit einem Schutzgruppenreagenz in die N-geschützte Form der Formel IX 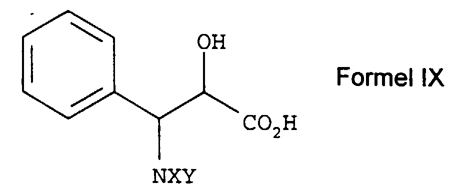 umwandelt, wobei X und Y die oben angegebene Bedeutung haben,
m) und anschließend nach üblichen Methoden mit einem Veresterungsreagenz zu Verbindungen der Formel I 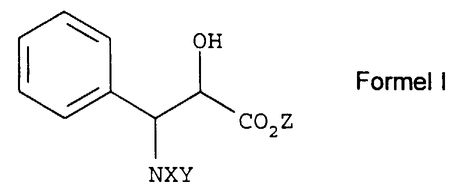 umsetzt, wobei X, Y und Z die oben angegebene Bedeutung haben.

2. Verfahren nach Anspruch 1,
**dadurch gekennzeichnet**,
daß man N-substituierte Phenylglycinol-Derivate der allgemeinen Formel V mit Hilfe von TEMPO (2,2,6,6-Tetramethylpiperidin-1-oxyl) und Hypochlorit-Lösung zu den korrespondierenden Phenylglycinal-Derivaten VI oxidiert, wobei X und Y die unter Anspruch 1 angegebene Bedeutung haben.

3. Verfahren nach Anspruch 1,
**dadurch gekennzeichnet**,
daß der Zusatz einer tertiären Stickstoffbase, wie z. B. Triethylamin im Bereich von 10 - 0.01 mol% die Cyanidierung des Aminoaldehyds mit Cyanwasserstoff begünstigt.

4. Verfahren nach Anspruch 1,
**dadurch gekennzeichnet,**
daß für die Cyanidierung tertiäre Stickstoffbasen, wie z. B. Triethylamin im Bereich von 0.1 - 0.05 mol% besonders bevorzugt sind.

5. Verfahren nach Anspruch 1,
**dadurch gekennzeichnet**,
daß die Bildung des Nitrils der allgemeinen Formel VII 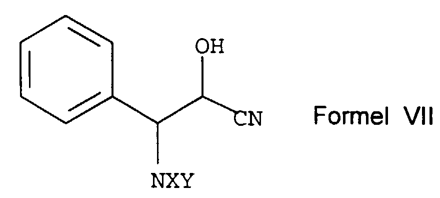 durch Umsetzung des entsprechenden Aminoaldehyds VI in Gegenwart eines organischen Lösungsmittels unter Zusatz von Cyanwasserstoff und einer Base mit Diastereoselektivitäten von > 74 : 26 erfolgt.

6. Verfahren nach Anspruch 1,
**dadurch gekennzeichnet**,
daß die Hydroxyaminosäure der allgemeinen Formel VIII und XI 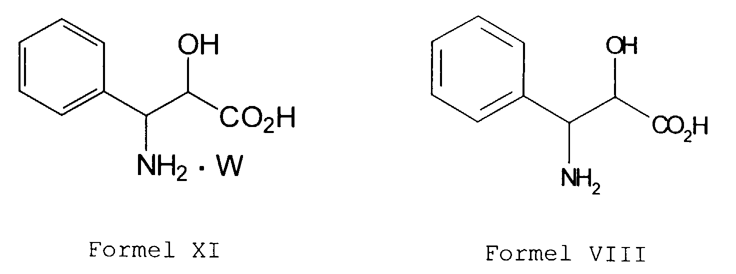 in Diastereoselektivitäten > 95 : 5 anfallen, indem man Verbindungen der Formel XI mit W = HCl nach Hydrolyse des entsprechenden Cyanhydrins der Formel VII einengt oder die Verbindung der Formel VIII, ebenfalls erhalten aus VII durch Hydrolyse, auf einen pH-Wert von 4 - 8 einstellt.

7. Verwendung von Verbindungen der allgemeinen Formel I hergestellt nach einem der vorhergehenden Ansprüche zur Herstellung von Taxolen.

8. Verwendung nach Anspruch 7, wobei die Verbindung der allgemeinen Formel I ein N-Benzoyl-3-amino-2-hydroxy-3-phenylpropionsäuremethylester ist.

## Claims

1. Process for the preparation of β-amino-α-hydroxy-carboxylic acids and derivatives having the general structure (2R,3S)- or (2S,3R)-N-(X,Y)-3-amino-2-hydroxy-3-phenylpropionic acid-Z corresponding to formula I 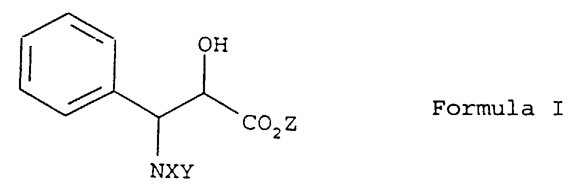 wherein
X denotes H, (C₁-C₆) alkyl, benzyl,
Y denotes (C₁-C₆) alkyl, benzyl, formyl, COR¹ or CO₂R²,
X,Y together denote phthaloyl, maleinoyl or maloneyl,
R¹ denotes (C₁-C₆) alkyl, phenyl, benzyl, NH₂, 4-NO₂-phenyl or 4-NO₂-benzyl,
R² denotes (C₁-C₆) alkyl, phenyl, benzyl, 4-NO₂-phenyl or 4-NO₂-benzyl,
Z denotes H, (C₁-C₅) alkyl, phenyl, benzyl, 4-NO₂-benzyl, 4-NO₂-phenyl or allyl,
characterised in that
a) (S)- or (R)-phenylglycine having the formula III 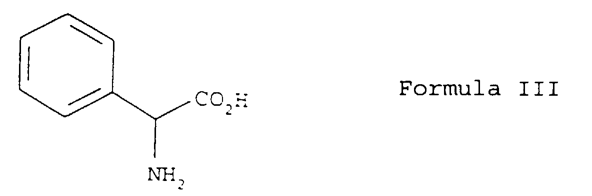 is reduced with hydride reagents,
b) the resulting (S)- or (R)-phenylglycinol having the formula IV 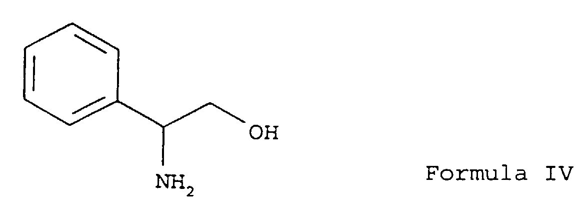 is converted by conventional methods into the
c) N-protected β-aminoalcohol (S)- or (R)-N-(X,Y)-phenylglycinol corresponding to the general formula V, 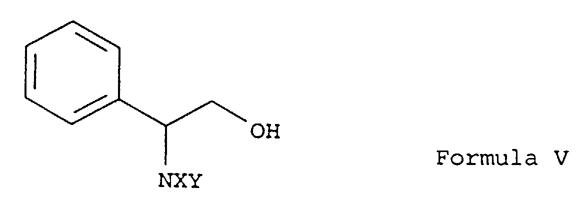 wherein X and Y have the meanings given above,
d) the (S)- or (R)-N-(X,Y)-phenylglycinal which is obtained from compounds corresponding to formula V by known oxidation methods and corresponds to formula VI, 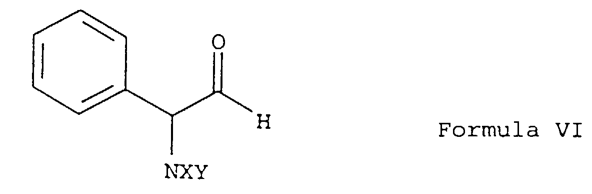 wherein X and Y have the meanings given above,
e) is converted in known per se manner into the (1RS,2S)- or (1RS,2R)-2-(X,Y)-amino-1-cyano-2-phenylethan-1-ol corresponding to formula VII, 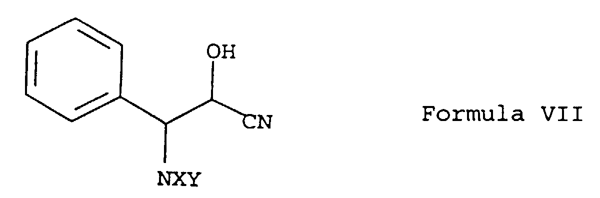 wherein X and Y have the meanings given above,
f) the nitrile obtained (formula VII) is hydrolysed by conventional methods to the acids VIII + IX or even to their addition salts corresponding to formulae X and XI, 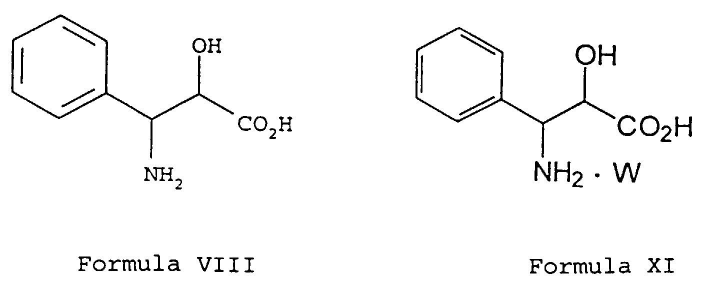 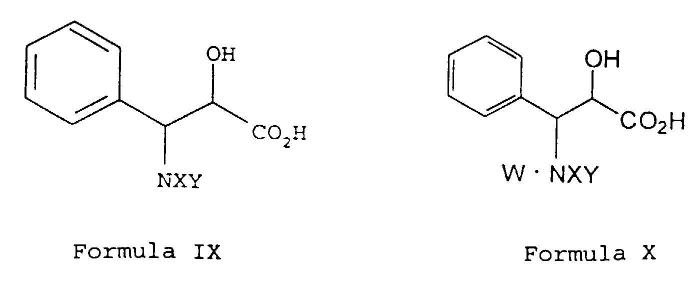 wherein X and Y have the meanings given above and W represents HCl, HBr or H₂SO₄,
g) the compounds corresponding to formulae VIII and XI are then converted by conventional methods into the (2RS,3S)- or (2RS,3R)-3-amino-2-hydroxy-3-phenylpropionic ester corresponding to formula XII, 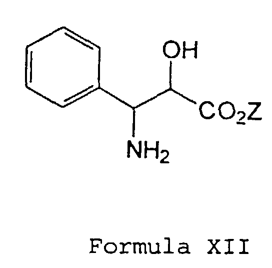 wherein Z equally denotes (C₁-C₆) alkyl, phenyl, benzyl, 4-NO₂-phenyl, 4-NO₂-benzyl or allyl, and
i) then the still free nitrogen function of formula X is protected by likewise known methods, which leads to compounds corresponding to formula I, 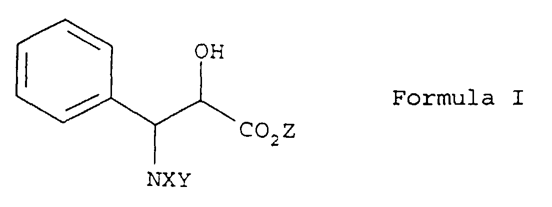 wherein Z, X and Y have the meanings given above,
or that
k) the compounds IX and X are converted as described under g) into compounds corresponding to formula I,
or that
l) the (2RS,3S)- or (2RS,3R)-3-amino-2-hydroxy-2-phenylpropionic acid having the formula VIII 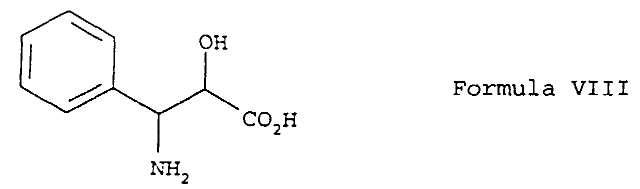 in conventional manner, by means of a protective-group reagent, is first of all changed into the N-protected form corresponding to formula IX, 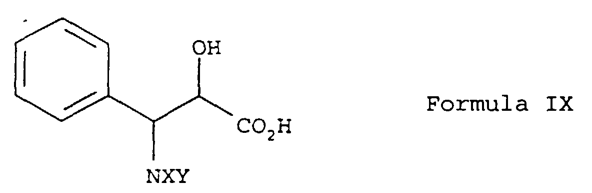 wherein X and Y have the meanings given above,
m) and subsequently converted by conventional methods, using an esterifying reagent, into compounds corresponding to formula I, 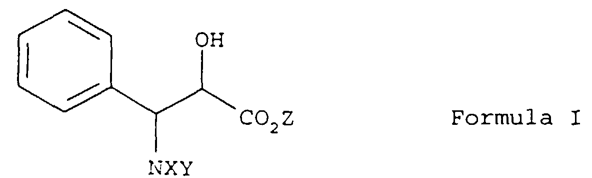 wherein X, Y and Z have the meanings given above.

2. Process according to claim 1,
characterised in that
N-substituted phenylglycinol derivatives corresponding to the general formula V are oxidised by means of TEMPO (2,2,6,6-tetramethyl-1-piperidinyloxy) and hypochlorite solution into the corresponding phenylglycinal derivatives VI, wherein X and Y have the meanings given in claim 1.

3. Process according to claim 1,
characterised in that
the addition of a tertiary nitrogen base such as, for example, triethylamine in the range of 10 to 0.01 mol.%, favours the cyanidation of the aminoaldehyde with hydrogen cyanide.

4. Process according to claim 1,
characterised in that
tertiary nitrogen bases such as, for example, triethylamine in the range of 0.1 to 0.05 mol.%, are particularly preferred for the cyanidation.

5. Process according to claim 1,
characterised in that
the formation of the nitrile corresponding to the general formula VII 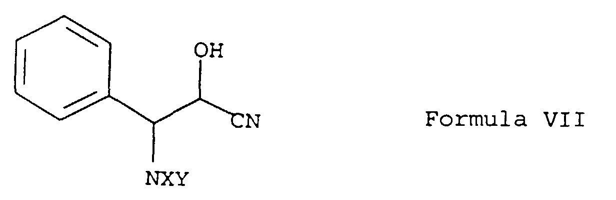 is effected through the conversion of the corresponding aminoaldehyde VI in the presence of an organic solvent with addition of hydrogen cyanide and of a base having diastereoselectivities of > 74 : 26.

6. Process according to claim 1,
characterised in that
the hydroxyamino acids corresponding to the general formulae VIII and XI 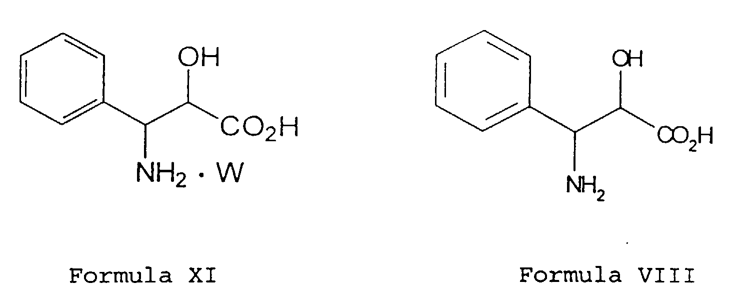 are obtained in diastereoselectivities of > 95 : 5, by concentrating to small volume compounds corresponding to formula XI, wherein W = HCl, after hydrolysis of the corresponding cyanohydrin having the formula VII, or by adjusting the compound having the formula VIII, likewise obtained from VII by hydrolysis, to a pH value of 4 to 8.

7. Use of compounds corresponding to the general formula I, prepared according to one of the preceding claims, for the production of Taxol.

8. Use according to claim 7, wherein the compound corresponding to the general formula I is a methyl N-benzoyl-3-amino-2-hydroxy-3-phenylpropionate.

## Revendications

1. Procédé d'obtention d'acides β-amino-α-hydroxycarboxyliques et des dérivés de structure générale (2R,3S)- ou (2S,3R)-N-(X,Y)-3-amino-2-hydroxy-3-phénylpropionique-Z de formule I 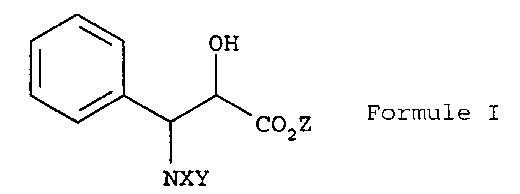 dans laquelle,
X signifie H, alkyle en C₁-C₆, benzyle
Y signifie alkyle en C₁-C₆, benzyle, formyle, COR¹, ou CO₂R²,
X, Y ensemble signifient phtaloyle, maléinoyle ou malonéyle,
R¹ signifie alkyle en C₁-C₆, phényle, benzyle, NH₂, 4-NO₂-phényle, ou 4-NO₂-benzyle,
R² signifie alkyle en C₁-C₆, phényle, benzyle, 4-NO₂-phényle, ou 4-NO₂-benzyle,
Z signifie H, alkyle en C₁-C₅, phényle, benzyle, 4-NO₂-benzyle, 4-NO₂-phényle ou allyle.
caractérisé en ce qu'
a) on réduit la (S)- ou (R)-phénylglycine de formule III, 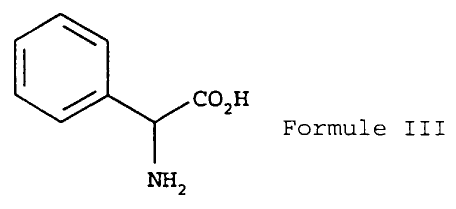 avec des réactifs du type hydride
b) on convertit le (S) ou (R) phénylglycinol de formule IV obtenu, 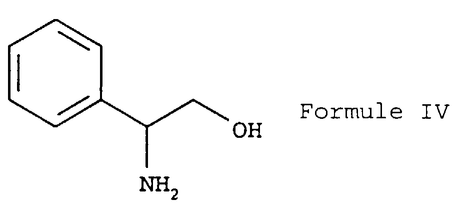 selon les méthodes usuelles en
c) β-aminoalcool-N-protégés (S)- ou (R)-N-(X,Y)-phénylglycinol de formule générale V, 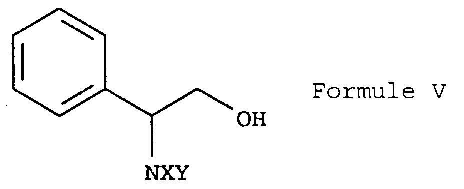 dans laquelle X et Y possèdent les significations mentionnées ci-dessus,
d) on convertit le (S)- ou (R)-N-(X, Y) phénylglycinal de formule VI obtenu, par les méthodes d'oxydation connues à partir des composés de formule V, 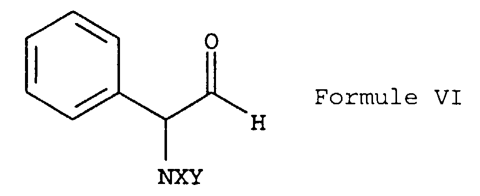 dans laquelle X et Y possèdent les significations citées ci-dessus,
e) on transforme d'une manière connue en soi en (1RS,2S)- ou en (1RS,2R)-2-(X,Y)-amino-1-cyano-2-phényléthane-1-ol de formule VII, 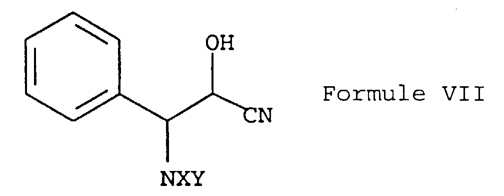 dans laquelle X et Y ont les significations mentionnées ci-dessus,
f) on hydrolyse le nitrile (formule VII) obtenu selon les méthodes usuelles, en acides VIII + IX ou en leurs sels d'addition de formule X et XI 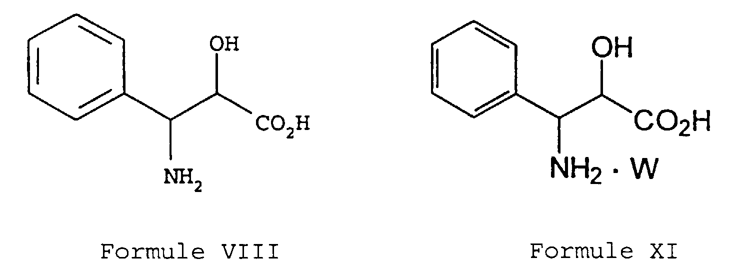 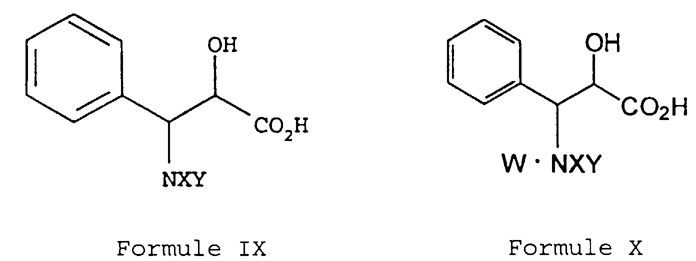 dans lesquelles X et Y possèdent les significations mentionnées ci-dessus et W représente ClH, BrH, ou SO₄H₂,
g) on transforme ensuite les composés de formule VIII et XI selon les méthodes usuelles en ester d'acide (2RS, 3S)-ou (2RS, 3R)-3-amino-2-hydroxy-3-phénylpropionique de formule XII, 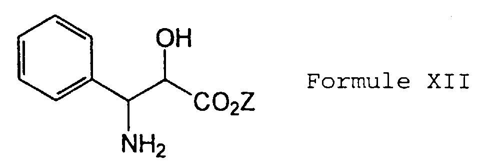 dans laquelle Z est égal à alkyle en C₁-C₆, phényle, benzyle, 4-NO₂-phényle, 4-NO₂-benzyle ou allyle,
et
i) ensuite on protège la fonction azotée encore libre du composé de formule X selon les méthodes pareillement connues, ce qui conduit aux composés de formule I, 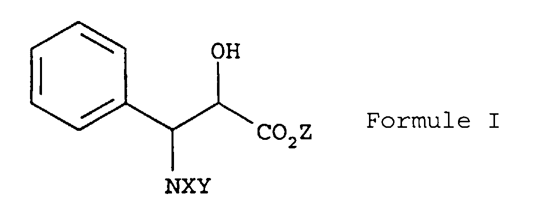 dans laquelle Z, X et Y possèdent les significations mentionnées ci-dessus, ou
k) on transforme les composés IX et X comme décrit en g), en composés de formule I, ou
l) on convertit l'acide (2RS, 3S) ou (2RS, 3R)-3-amino-2-hydroxy-2-phénylpropionique de formule VIII, 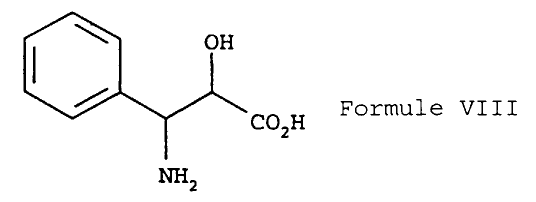 en premier lieu d'une manière usuelle, avec un réactif à groupe protecteur, en la forme N-protégée de formule IX, 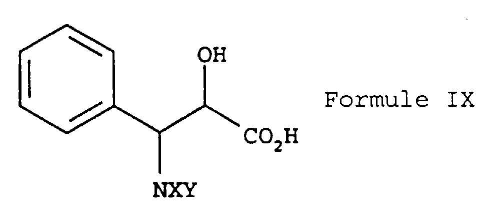 dans laquelle X et Y possèdent les significations mentionnées ci-dessus, et
m) on transforme ensuite selon les méthodes usuelles avec un réactif d'estérification en composés de formule I, 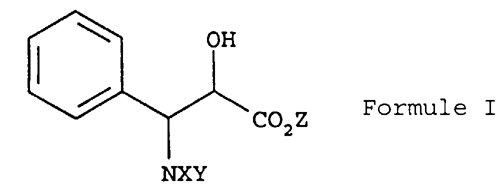 dans laquelle X, Y et Z possèdent les significations mentionnées ci-dessus.

2. Procédé selon la revendication 1,
caractérisé en ce qu'
on oxyde les dérivés N-substitués du phénylglycinol de formule générale V à l'aide de TEMPO (2,2,6,6-tétraméthylpiperidin-1-oxyl) et de solution d'hypochlorite, en phénylgycinal dérivés correspondants VI, dans laquelle X et Y possèdent les significations indiquées à la revendication 1.

3. Procédé selon la revendication 1,
caractérisé en ce que
l'addition d'une base azotée tertiaire comme par exemple la triéthylamine dans la plage de 10 à 0,01 % molaire, favorise la cyanuration de l'aminoaldéhyde avec l'acide cyanhydrique.

4. Procédé selon la revendication 1,
caractérisé en ce que
pour la cyanuration, des bases azotées tertiaires comme par exemple la triéthylamine dans la plage de 0,1 à 0,05 % molaire sont particulièrement préférées.

5. Procédé selon la revendication 1,
caractérisé en ce que
la formation du nitrile de formule générale VII, 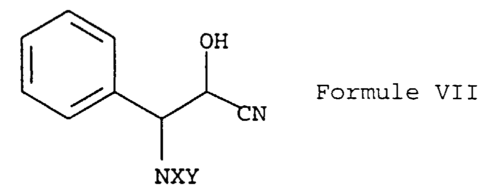 s'effectue par transformation de l'aminoaldéhyde VI correspondant en présence d'un solvant organique en ajoutant de l'acide cyanhydrique et une base, avec des sélectivités en diastéréoisomères de > 74 : 26.

6. Procédé selon la revendication 1,
caractérisé en ce que
les hydroxyaminoacides de formules générales VIII et XI 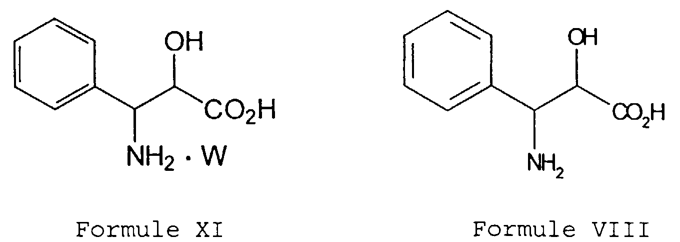 se produisent avec une diastéréosélectivité > 95 : 5, procédé dans lequel on concentre les composés de formule XI avec W = ClH après hydrolyse de la cyanhydrine correspondante de formule VII, ou dans lequel on ajuste le composé de formule VIII, également obtenu à partir de VII par hydrolyse, à une valeur de pH de 4 à 8.

7. Utilisation de composés de formule générale I préparés selon l'une des revendications précédentes, en vue de la préparation des taxols.

8. Utilisation selon la revendication 7, dans laquelle le composé de formule générale I, est un ester méthylique d'acide N-benzoyl-3-amino-2-hydroxy-3-phénylpropionique.
